# EUROPÄISCHE PATENTSCHRIFT

(11) **EP 1 878 395 B1**
(45) Veröffentlichungstag und Bekanntmachung des Hinweises auf die Patenterteilung: **13.01.2010**
(21) Anmeldenummer: 07013544.7
(22) Anmeldetag: 11.07.2007
(51) Int. Cl.: A61B 17/28

(54) **Medizinisches Instrument**
Medical instrument
Instrument médical

(30) Priorität: 11.07.2006 DE 102006031971
(43) Veröffentlichungstag der Anmeldung: 16.01.2008
(73) Patentinhaber: Karl Storz GmbH & Co. KG, 78532 Tuttlingen (DE)
(72) Erfinder: Summerer,Sabine, 85551 Kirchheim (DE)
(74) Vertreter: Hofmeister, Frank

(56) Entgegenhaltungen:
- WO-A-02/071956
- DE-A1- 19 813 781
- US-A- 5 906 630
- US-A- 5 919 206

## Beschreibung

Die Erfindung betrifft ein medizinisches Instrument mit einem Schaft, an dessen proximalem Ende eine aus zwei Griffteilen bestehende Handhabe angeordnet ist und an dessen distalem Ende ein aus zwei Maulteilen bestehendes Werkzeug angeordnet ist, wobei zum Öffnen und Schließen des Werkzeugs mindestens ein Maulteil über eine Zug-/Druckstange gegenüber dem anderen Maulteil um einen von der Stellung der Maulteile zueinander abhängigen variablen virtuellen Drehpunkt verschwenkbar ist, und wobei die Zug-/Druckstange proximalseitig mit einem verschwenkbar ausgebildeten Griffteil der Handhabe verbunden ist.

Gattungsgemäße medizinische Instrumente werden in der Praxis häufig als Greif-, Halte- und/oder Schneidwerkzeuge eingesetzt. So können die Maulteile Schneiden aufweisen, um Gewebe abzutrennen, oder stumpfe Flächen aufweisen, um beispielsweise abgetrenntes Gewebe zu halten, oder Blutgefäße abzuklemmen.

Diesen medizinischen Instrumenten ist gemeinsam, dass beide Maulteile des am distalen Ende des Schaftes angeordneten Werkzeugs um einen gemeinsamen Drehpunkt verschwenkbar sind. Zum Öffnen und Schließen der Maulteile ist eine Zug-/Druckstange vorgesehen, die mit einem beweglichen Griffteil der Handhabe gekoppelt ist.

Ein solches gattungsgemäßes medizinisches Instrument ist beispielsweise aus der DE 299 11 011 U bekannt. Bei diesem bekannten medizinischen Instrument liegen die Anlenkpunkte der Gelenkhebel an den jeweiligen Maulteilen weit entfernt von dem gemeinsamen Drehpunkt der Maulteile, damit sich beim Verschieben der Zug/Druckstange, insbesondere zum Schließen des Werkzeugs, ein Hebelverhältnis einstellt, dass eine große Kraftübertragung ermöglicht. Diese medizinischen Instrumente haben sich in der Praxis bewährt, jedoch weisen sie unter beengten Platzverhältnissen, wie beispielsweise in der endoskopischen Chirurgie, den Nachteil auf, dass die Gelenkhebel beim Öffnen der Maulteile nach außen schwenken und so im Bereich zwischen Zug-/Druckstange und Werkzeug den Durchmesser des Instruments deutlich vergrößern. Dieser Platzbedarf steht aber nicht immer zur Verfügung, weshalb diese bekannten Instrumente nicht bei allen Operationen eingesetzt werden können.

Die US-A-5 919 206" von der die Präambel der unabhängigen Anspruche abgeleitet wird, offenbart ein gattungsgemäßes Instrument, dessen mindestens ein Maulteil über eine Zug-/Druckstange gegenüber dem anderen Maulteil um einen Drehpunkt (16) verschwenkbar ist. Der Drehpunkt ist dabei als von der Stellung der Maulteile zueinander abhängiger variabler virtueller Drehpunkt ausgebildet.

Davon ausgehend liegt der Erfindung die **Aufgabe** zugrunde, ein medizinisches Instrument der eingangs genannten Art so auszugestalten, dass dieses auch unter beengten Platzverhältnissen und mit ausreichender Kraftübertragung einsetzbar ist.

Diese Aufgabe ist erfindungsgemäß durch ein medizinisches Instrument mit den Merkmalen des unabhängigen Anspruchs 1 oder des unabhängigen Anspruchs 5 gelöst.
Die abhängigen Ansprüche beinhalten vorteilhafte Weiterbildungen der Erfindung.

Durch die Ausbildung des Drehpunktes als von der Maulteilstellung abhängiger variabler virtueller Drehpunk wird die von der Instrumentenlängsachse fort nach außen weisende Radialbewegung der Gelenkmechanik der Maulteile im Bereich des Drehpunktes so verringert, dass die Gelenkmechanik zum Ansteuern der Maulteile unabhängig von der Stellung der Maulteile zueinander immer innerhalb des Instrumentendurchmessers liegt.

Gemäß einer ersten praktischen Ausführungsform der Erfindung wird vorgeschlagen, dass beide Maulteile verschwenkbar ausgebildet auf zwei quer zur Instrumentenlängsachse im Schaft gelagerten Achsen gelagert sind. Durch die Lagerung der Maulteile auf zwei im Schaft gelagerten Achsen kann die Radialbewegung der Maulteile im Bereich des Drehpunktes deutlich verringert werden.

Das Koppeln der verschwenkbaren Maulteile mit den im Schaft gelagerten Achsen erfolgt erfindungsgemäß vorteilhafterweise über jeweils zwei Anlenkhebel pro Maulteil, wobei die Anlenkhebel für ein erhöhtes Kraftübersetzungsverhältnis zwischen Zug-/Druckstange und den Maulteilen sorgen, verglichen mit den Instrumenten des Standes der Technik.

Mit einer bevorzugten Ausführungsform der Erfindung wird vorgeschlagen, dass jeder Anlenkhebel eines jeden Maulteils mit einem Ende auf einer der Achsen gelagert ist und mit dem anderen Ende auf einem ortsfest im jeweiligen Maulteil angeordneten Zapfen verschwenkbar gelagert ist. Aufgrund der beidseitig verschwenkbaren Lagerung der Anlenkhebel verlagern sich die Anlenkhebel eines jeden Maulteils in Abhängigkeit von der jeweiligen Arbeitsstellung der Maulteile relativ zueinander so, dass eine jeweils bestmögliche Kraftübertragung von der Zug-/Druckstange auf die Maulteile gewährleistet ist.

Weiterhin wird mit der Erfindung vorgeschlagen, dass der Schnittpunkt der Mittelachsen der Anlenkhebel eines Maulteils den Drehpunkt der Maulteile bildet. Dieser sich je nach Stellung der Maulteile zueinander verlagernde virtuelle Drehpunkt bewirkt, dass die Gelenkmechanik zum Ansteuern der Maulteile unabhängig von der Stellung der Maulteile zueinander immer innerhalb des Instrumentendurchmessers liegt, weshalb ein solchermaßen ausgebildetes medizinisches Instrument auch bei beengten Platzverhältnissen, wie beispielsweise in der endoskopischen Chirurgie einsetzbar ist.

Gemäß einer bevorzugten Ausführungsform der Erfindung wird vorgeschlagen, dass in den Maulteilen Freischnitte für die Achsen ausgebildet sind, damit die Achsen, auf denen die Anlenkhebel gelagert sind, die Maulteile durchdringen, ohne Einfluss auf die Maulteile zu nehmen, und die über die Anlenkhebel auf den Achsen gelagerten Maulteile beim Verschwenken mittels der Zug-/Druckstange positionsgenau in die jeweilige Arbeitsstellung überführbar sind.

Mit einer zweiten praktischen Ausführungsform der Erfindung wird vorgeschlagen, dass beide Maulteile über jeweils zwei Anlenkhebel verschwenkbar ausgebildet sind, wobei jeder Anlenkhebel eines jeden Maulteils verschwenkbar mit einem Ende ortsfest auf einem am jeweiligen Maulteil angeordneten Zapfen gelagert ist und mit dem anderen Ende auf einem ortsfest auf der diesem Maulteil benachbarten Seite des Schaftes angeordneten Achsstummel verschwenkbar gelagert ist.

Bei dieser Ausgestaltungsform bildet der Schnittpunkt der Mittelachsen der Anlenkhebel eines Maulteils den variablen und von der jeweiligen Maulteilstellung abhängigen Drehpunkt der Maulteile.

Gemäß einer weiteren erfindungsgemäßen Ausführungsform wird vorgeschlagen, dass beide Maulteile über jeweils einen Anlenkhebel verschwenkbar ausgebildet sind, wobei jeder Anlenkhebel eines jeden Maulteils verschwenkbar mit einem Ende ortsfest auf einem am jeweiligen Maulteil angeordneten Zapfen gelagert ist und mit dem anderen Ende verschwenkbar auf einem ortsfest auf der diesem Maulteil benachbarten Seite des Schaftes angeordneten Achsstummel gelagert ist.

Schließlich wird mit der Erfindung vorgeschlagen, dass die Zug-/Druckstange des vorzugsweise für endoskopische Zwecke verwendbaren medizinischen Instruments distalseitig über jeweils einen Gelenkhebel mit jedem verschwenkbaren Maulteil verbunden ist. Die Verwendung der Gelenkhebel stellt eine konstruktiv einfache Ausgestaltungsform dar, um bei guter Kraftübertragung auf die Maulteile ausgehend von einer Zug-/Druckstange zwei Maulteile verschwenken zu können.

Weiterhin wird mit der Erfindung vorgeschlagen, dass in jedem Maulteil eine Führungsbahn zur Aufnahme eines an der benachbarten Seite des Schaftes angeordneten Lagerstiftes ausgebildet ist, um das führende Verschwenken der Maulteile zu erleichtern.

Der variable Drehpunkt der Maulteile wird bei dieser Ausgestaltungsform durch den Schnittpunkt der Mittelachse des Anlenkhebels eines Maulteils mit der Führungsbahn gebildet.

Zur Ausbildung des Instrumentenschaftes wird mit der Erfindung weiterhin vorgeschlagen, dass das distale Ende des Schaftes zwei im Wesentlichen parallel zueinander verlaufende Stege umfassend gabelförmig ausgebildet ist und die proximalen Enden der Maulteile zwischen den Stegen gelagert sind. Diese konstruktive Ausgestaltung des Instrumentenschaftes erleichtert die Montage und Demontage des Instrumentes zu Reinigungszwecken. Vorteilhafterweise sind die Achsen, auf denen die Maulteile führend gelagert sind, im Bereich der Stege im Schaft gelagert.

Weitere Merkmale und Vorteile der Erfindung ergeben sich anhand der zugehörigen Zeichnung, in der drei Ausführungsbeispiele eines erfindungsgemäßen medizinischen Instruments nur beispielhaft dargestellt sind, ohne die Erfindung auf diese Ausführungsbeispiele zu beschränken. In der Zeichnung zeigt:
- Fig. 1: eine Seitenansicht eines erfindungsgemäßen medizinischen Instruments;
- Fig. 2: eine vergrößerte Ansicht des Details II gemäß Fig. 1 eine erste Ausführungsform darstellend;
- Fig. 3: einen Schnitt entlang der Linie III-III gemäß Fig. 2;
- Fig. 4: eine Ansicht gemäß Fig. 2, das medizinische Instrument jedoch ohne den Schaft darstellend;
- Fig. 5: eine Seitenansicht des unteren Maulteils gemäß Fig. 4;
- Fig. 6a: eine Ansicht gemäß Fig. 4, die Maulteile im geschlossenen Zustand darstellend;
- Fig. 6b: eine Ansicht gemäß Fig. 6a, jedoch die Maulteile im teilweise geöffneten Zustand darstellend;
- Fig. 6c: eine Ansicht gemäß Fig. 6a, jedoch die Maulteile im vollständig geöffneten Zustand darstellend.:
- Fig. 7a: eine Ansicht gemäß Fig. 4, jedoch eine zweite erfindungsgemäße Ausführungsform darstellend;
- Fig. 7b: eine Ansicht gemäß Fig. 7a, jedoch die Maulteile im geöffneten Zustand darstellend;
- Fig. 7c: einen Schnitt entlang der Linie VIIc-VIIc gemäß Fig. 7a;
- Fig. 8a: eine Ansicht gemäß Fig. 4, jedoch eine dritte erfindungsgemäße Ausführungsform darstellend und
- Fig. 8b: eine Ansicht gemäß Fig. 8a, jedoch die Maulteile im geöffneten Zustand darstellend.

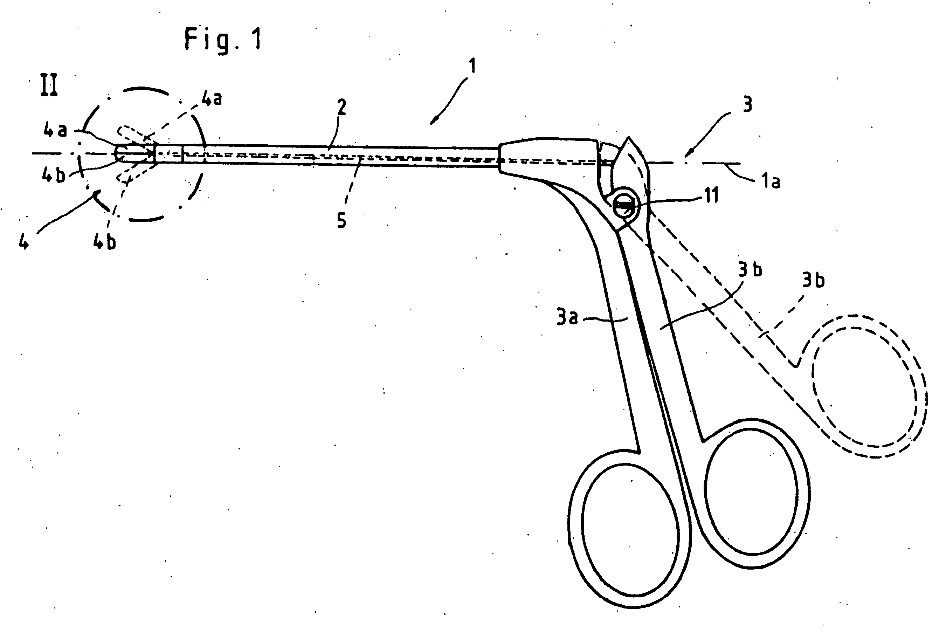

Die Abbildung Fig. 1 zeigt die Seitenansicht eines medizinischen Instruments, dessen Kraftübertragungsmechanismus vielseitig verwendet werden kann, wie beispielsweise für Stanzen, Scheren, Nadelhalter, Fassinstrumente und dergleichen.

Das nur exemplarisch dargestellte medizinische Instrument 1 besteht im Wesentlichen aus einem hohlen Schaft 2, an dessen proximalem Ende eine Handhabe 3 angeordnet ist, die aus einem starren Griffteil 3a und einem gegenüber dem starren Griffteil 3a verschwenkbaren Griffteil 3b besteht. Am distalen Ende des Schaftes 2 ist ein Werkzeug 4 angeordnet, das von zwei verschwenkbaren Maulteilen 4a und 4b gebildet wird.

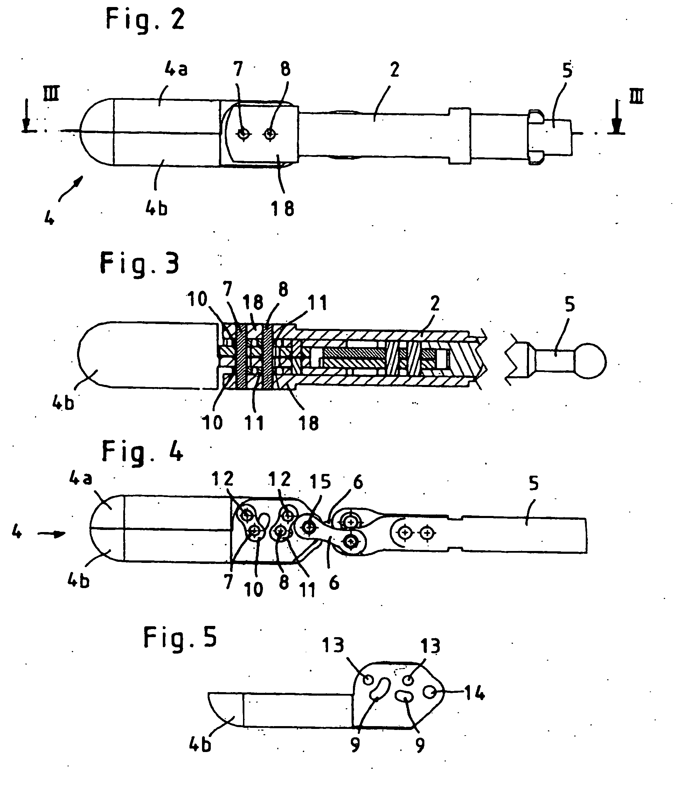

Wie insbesondere aus den Detailansichten gemäß den Abbildungen Fig. 3, 4 und 6a bis 10b ersichtlich, sind die Maulteile 4a, 4b des Werkzeugs 4 und der verschwenkbare Griffteil 3b der Handhabe 3 über eine in dem hohlen Schaft 2 gelagerte Zug-/Druckstange 5 so miteinander gekoppelt, dass durch das Verstellen des Griffteils 3b der Handhabe 3 die Maulteile 4a und 4b des Werkzeugs 4 von der geschlossenen Stellung (durchgezogene Darstellung in Fig. 1 sowie Fig. 4, 6a, 7a, 8a, 9a und 10a) in die geöffnete Stellung (gestrichelte Darstellung in Fig. 1 sowie Fig. 6b, 6c, 7b und 8b) bzw. umgekehrt überführbar sind. Die jeweils zugehörige Stellung des verschwenkbaren Griffteils 3b ist in der Abbildung Fig. 1 ebenfalls durchgezogen (für die geschlossene Stellung) und gestrichelt (für eine geöffnete Stellung) dargestellt.

Den Abbildungen Fig. 4 und 6a bis 7c ist weiterhin zu entnehmen, dass bei diesen drei Ausgestaltungsformen die Zug-/Druckstange 5 nicht unmittelbar, sondern unter Zwischenschaltung zweier Gelenkhebel 6 mit den Maulteilen 4a und 4b verbunden ist. Die Gelenkhebel 6 ermöglichen eine gute Kraftübertragung von der Zug-/Druckstange 5 auf die beiden Maulteile 4a und 4b.

Um sicherzustellen, dass bei der in den Abbildungen Fig. 2 bis 6c dargestellten ersten Ausführungsform einerseits eine ausreichende Kraftübertragung für die aufzubringenden Schneid- oder Klemmkräfte von der Zug-/Druckstange 5 auf die Maulteile 4a und 4b aufbringbar ist und andererseits die Abmessungen des Instruments 1 durch den Hebelmechanismus nicht vergrößert werden, sind die Maulteile 4a und 4b führend auf zwei Achsen 7 und 8 gelagert, die ihrerseits parallel zueinander und quer zur Instrumentenlängsachse 1a im Schaft 2 gelagert sind, wobei in den Maulteilen 4a und 4b bogenförmige Freischnitte 9 zum berührungsfreien Durchführen für die Achsen 7 und 8 ausgebildet sind.

Das Koppeln der verschwenkbaren Maulteile 4a und 4b mit den im Schaft 2 gelagerten Achsen 7 und 8 erfolgt über jeweils zwei Anlenkhebel 10 und 11 pro Maulteil 4a, 4b, wobei die Anlenkhebel 10, 11 für ein erhöhtes Kraftübersetzungsverhältnis zwischen Zug-/Druckstange 5 und den Maulteilen 4a, 4b sorgen.

Der konstruktive Aufbau der Maulteile 4a, 4b sowie deren Kopplung mit den Anlenkhebeln 10 und 11 ergibt sich insbesondere aus der Zusammenschau der Abbildungen Fig. 4 und 5. Jeder Anlenkhebel 10, 11 eines jeden Maulteils 4a, 4b ist mit einem Ende auf einer der Achsen 7, 8 gelagert ist und mit dem anderen Ende verschwenkbar auf einem ortsfest im jeweiligen Maulteil 4a, 4b angeordneten Zapfen 12 gelagert. Dem in Fig. 5 in Alleinstellung dargestellten Maulteil 4b sind die verschiedenen Lagerpunkte für die Anlenkhebel 10 und 11 sowie die Gelenkhebel 6 zu entnehmen, nämlich Lagerbohrungen 13 zur Aufnahme der Zapfen 12, die bogenförmigen Freischnitte 9 für die Achsen 7 und 8 sowie eine Bohrung 14 zur Aufnahme eines Lagerzapfens 15 am distalseitigen Ende des Gelenkhebels 6.

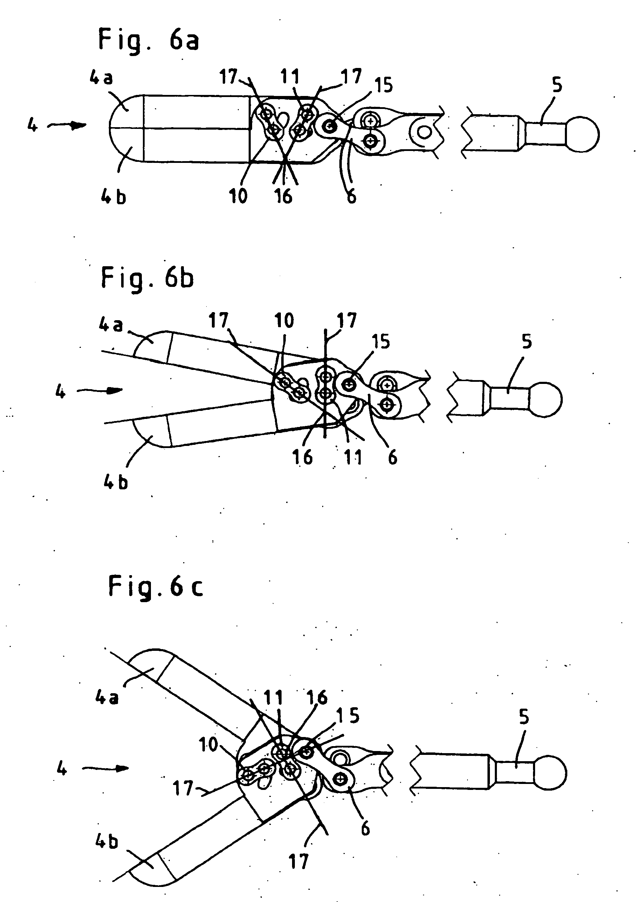

Die Arbeitsweise eines solchermaßen ausgebildeten medizinischen Instruments 1 ist in den Abbildungen Fig. 6a bis 6c dargestellt, die die Maulteile 4a und 4b in drei verschiedenen Arbeitspositionen zeigen.

Aufgrund der Lagerung der Maulteile 4a und 4b über die Anlenkhebel 10 und 11 auf den beiden parallel zueinander angeordneten Achsen 7 und 8 ergibt sich kein ortsfester Drehpunkt, um den sich die beiden Maulteile 4a und 4b ausgehend von der in Fig. 6a dargestellten geschlossenen Position hin zur vollständig geöffneten Position gemäß Fig. 6c drehen. Der Drehpunkt der Maulteile 4a und 4b ergibt sich bei der dargestellten ersten konstruktiven Ausgestaltungsform aus dem Schnittpunkt 16 der beiden Mittelachsen 17 der Anlenkhebel 10 und 11 eines jeden Maulteils 4a, 4b.

Wie aus den Darstellungen Fig. 6a bis 6c ersichtlich, ergibt sich aus dieser Konstruktion ein variabler Drehpunkt für die Maulteile 4a, 4b, so dass unabhängig von der Stellung der Maulteile 4a, 4b zueinander die Gelenkmechanik zum Ansteuern der Maulteile 4a und 4b immer innerhalb des Instrumentendurchmessers liegt, weshalb ein derartig ausgebildetes medizinisches Instrument 1 auch bei beengten Platzverhältnissen, wie beispielsweise in der endoskopischen Chirurgie, einsetzbar ist.

Das distale Ende des Schaftes 2 ist bei der in Fig. 2 und 3 dargestellten Ausführungsform aus zwei im Wesentlichen parallel zueinander verlaufenden Stegen 18 bestehend gabelförmig ausgebildet, wobei die proximalen Enden der Maulteile 4a, 4b zwischen den Stegen 18 auf den Achsen 7 und 8 gelagert sind. Diese konstruktive Ausgestaltung des Instrumentenschaftes 2 erleichtert die Montage und Demontage des Instrumentes 1 zu Reinigungszwecken.

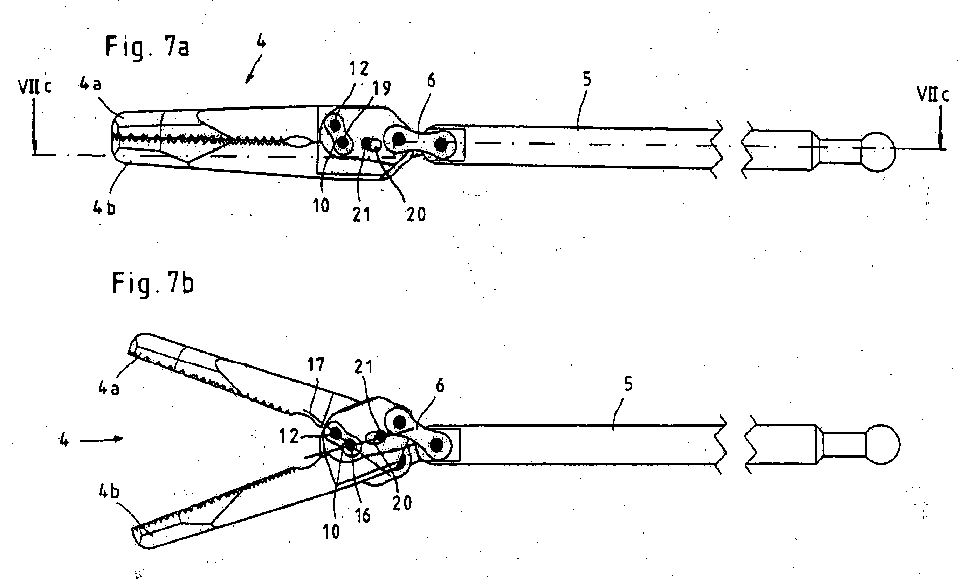

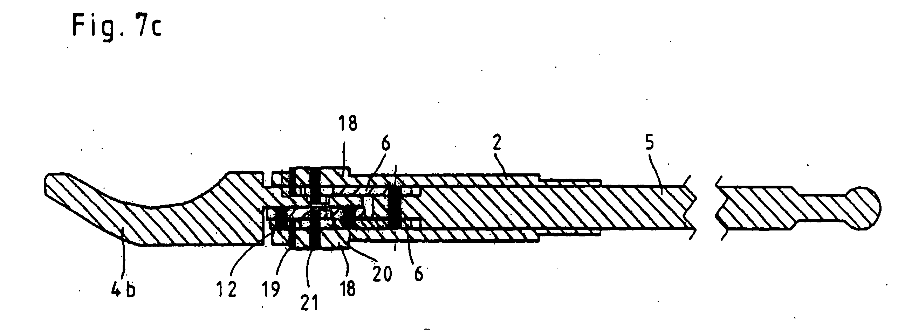

Die in den Abbildungen Fig. 7a bis 7c dargestellte zweite Ausführungsform des Kraftübertragungsmechanismus zwischen der Zug/Druckstange 5 und den Maulteilen 4a und 4b unterscheidet sich von der zuvor beschriebenen Ausführungsform dadurch, dass bei dieser Ausgestaltungsform jedes der beiden Maulteile 4a und 4b über jeweils nur einen Anlenkhebel 10 mit dem Schaft 2 gekoppelt ist.

Diese Kopplung erfolgt bei dieser Ausführungsform derart, dass jeder Anlenkhebel 10 eines jeden Maulteils 4a, 4b verschwenkbar mit einem Ende ortsfest auf einem am jeweiligen Maulteil 4a, 4b angeordneten Zapfen 12 gelagert ist und mit dem anderen Ende verschwenkbar auf einem ortsfest auf der diesem Maulteil 4a, 4b benachbarten Seite des Schaftes 2 angeordneten Achsstummel 19 gelagert ist.

Alternativ zu der einseitigen Lagerung der Anlenkhebel 10 auf dem Achsstummel 19 ist es auch möglich, die Anlenkhebel 10 auf durchgehenden Achsen zu lagern, wobei bei dieser Ausführungsform in den Maulteilen 4a und 4b Freischnitte für die Achsen vorzusehen sind.

Weiterhin ist in jedem Maulteil 4a, 4b eine Führungsbahn 20 zur Aufnahme eines an der benachbarten Seite des Schaftes 2 angeordneten Lagerstiftes 21 ausgebildet, über den das Verschwenken der Maulteile 4a und 4b unterstützt wird. Den Drehpunkt 16 der Maulteile 4a, 4b bildet bei dieser Ausgestaltungsform der Schnittpunkt 16 der Mittelachse 17 des Anlenkhebels 10 eines Maulteils 4a, 4b mit der Führungsbahn 20.

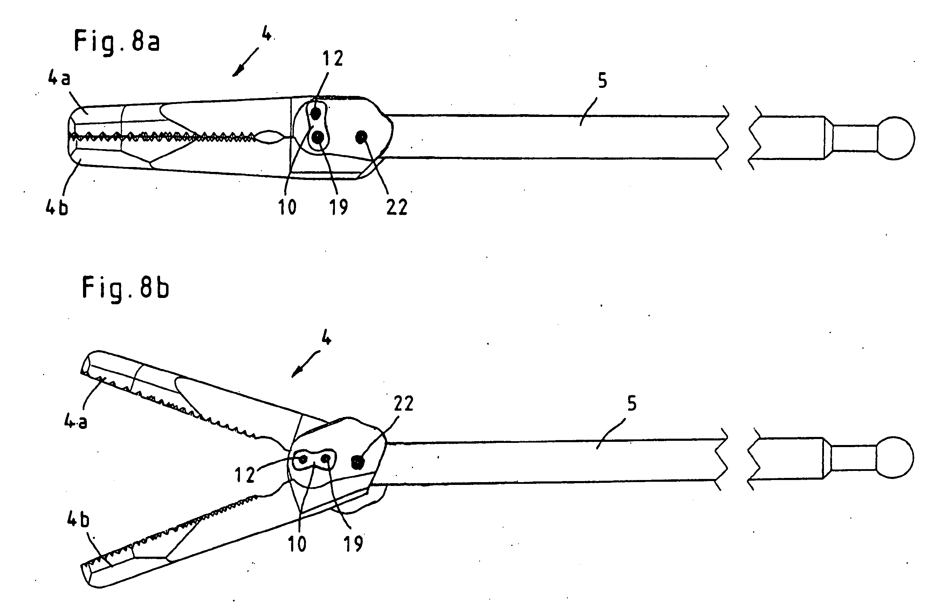

In den Abbildungen Fig. 8a und 8b ist eine dritte Ausführungsform des Kraftübertragungsmechanismus zwischen der Zug/Druckstange 5 und den Maulteilen 4a und 4b dargestellt. Diese Ausführungsform weist gegenüber den zwei zuvor beschriebenen konstruktiven Ausgestaltungen den wesentlichen Unterschied auf, dass die Zug-/Druckstange 5 nicht unter Zwischenschaltung von Gelenkhebeln 6, sondern direkt über einen Lagerbolzen 22 mit den Maulteilen 4a und 4b verbunden ist.

Die in den Abbildungen Fig. 8a und 8b dargestellte dritte Ausführungsform unterscheidet sich von der zuvor anhand der Abbildungen Fig. 7a bis 7c beschrieben Ausgestaltungsform weiterhin dadurch, dass hier in den Maulteilen 4a und 4b keine Führungsbahnen für Lagerstifte vorgesehen sind. Statt dessen erfolgt die Kopplung der verschwenkbaren Maulteile 4a, 4b mit dem Schaft 2 allein über jeweils einen Anlenkhebel 10, der mit einem Ende verschwenkbar, aber ortsfest auf einem am jeweiligen Maulteil 4a, 4b angeordneten Zapfen 12 gelagert ist und mit dem anderen Ende verschwenkbar auf einem ortsfest auf der diesem Maulteil 4a, 4b benachbarten Seite des Schaftes 2 angeordneten Achsstummel 19 gelagert ist.

Bei beiden Ausgestaltungsformen erfolgt die Kopplung der Maulteile 4a und 4b mit dem Schaft 2 über jeweils einen Achsstummel, der das jeweilige Maulteil 4a, 4b mit der benachbarten Seite des Schaftes 2 lagernd verbindet.

Alternativ zu der einseitigen Lagerung der Anlenkhebel 10 auf dem Achsstummel 19 ist es auch möglich, die Anlenkhebel 10 auf durchgehenden Achsen zu lagern, wobei bei dieser Ausführungsform in den Maulteilen 4a und 4b Freischnitte für die Achsen vorzusehen sind.

In der in Fig. 8a dargestellten Position mit geschlossenen Maulteilen 4a, 4b verbindet der Anlenkhebel 10 den Schaft 2, mit dem er mit dem unteren Drehpunkt (Zapfen 19) verbunden ist, mit dem jeweiligen Maulteil 4a, 4b, mit der Anlenkhebel 10 über den oberen Drehpunkt (Zapfen 12) verbunden ist.

In der in Fig. 8b dargestellten Position mit geöffneten Maulteilen 4a, 4b verbindet der Anlenkhebel 10 den Schaft 2, mit dem er mit dem proximalen Drehpunkt (Zapfen 19) verbunden ist, mit dem jeweiligen Maulteil 4a, 4b, mit der Anlenkhebel 10 folglich über den distalen Drehpunkt (Zapfen 12) verbunden ist. Somit bewegt sich der Drehpunkt bei dieser Ausgestaltungsform in etwa auf einem Viertelkreis.

### Bezugszeichenliste

| | | | |
|---|---|---|---|
| 1 | medizinisches Instrument | 13 | Lagerbohrung |
| 1a | Instrumentenlängsachse | 14 | Bohrung |
| 2 | Schaft | 15 | Lagerzapfen |
| 3 | Handhabe | 16 | Schnittpunkt/Drehpunkt |
| 3a | starrer Griffteil | 17 | Mittelachse |
| 3b | verschwenkbarer Griffteil | 18 | Steg |
| 4 | Werkzeug | 19 | Achsstummel |
| 4a | Maulteil | 20 | Führungsbahn |
| 4b | Maulteil | 21 | Lagerstift |
| 5 | Zug-/Druckstange | 22 | Lagerbolzen |
| 6 | Gelenkhebel | | |
| 7 | Achse | | |
| 8 | Achse | | |
| 9 | Freischnitt | | |
| 10 | Anlenkhebel | | |
| 11 | Anlenkhebel | | |
| 12 | Zapfen | | |

## Patentansprüche

1. Medizinisches Instrument mit einem Schaft (2), an dessen proximalem Ende eine aus zwei Griffteilen (3a, 3b) bestehende Handhabe (3) angeordnet ist und an dessen distalem Ende ein aus zwei Maulteilen (4a, 4b) bestehendes Werkzeug (4) angeordnet ist, wobei zum Öffnen und Schließen des Werkzeugs (4) mindestens ein Maulteil (4a oder 4b) über eine Zug-/Druckstange (5) gegenüber dem anderen Maulteil (4b oder 4a) um einen von der Stellung der Maulteile (4a, 4b) zueinander abhängigen variablen virtuellen Drehpunkt (16) verschwenkbar ist, und wobei die Zug-/Druckstange (5) proximalseitig mit einem verschwenkbar ausgebildeten Griffteil (3b) der Handhabe (3) verbunden ist,
**dadurch gekennzeichnet,**
**dass** beide Maulteile (4a, 4b) verschwenkbar ausgebildet auf zwei quer zur Instrumentenlängsachse (1a) im Schaft (2) gelagerten Achsen (7, 8) gelagert sind und jedes Maulteil (4a, 4b) über jeweils zwei Anlenkhebel (10, 11) verschwenkbar mit den Achsen (7, 8) gekoppelt ist.

2. Medizinisches Instrument nach Anspruch 1, **dadurch gekennzeichnet, dass** jeder Anlenkhebel (10, 11) eines jeden Maulteils (4a, 4b) mit einem Ende auf einer der Achsen (7, 8) gelagert ist und mit dem anderen Ende verschwenkbar auf einem ortsfest im jeweiligen Maulteil (4a, 4b) angeordneten Zapfen (12) gelagert ist.

3. Medizinisches Instrument nach Anspruch oder 2, **dadurch gekennzeichnet, dass** der Schnittpunkt (16) der Mittelachsen (17) der Anlenkhebel (10,11) eines Maulteils (4a, 4b) den Drehpunkt der Maulteile (4a, 4b) bildet.

4. Medizinisches Instrument nach einem der Ansprüche 1 bis 3, **dadurch gekennzeichnet, dass** in den Maulteilen (4a, 4b) Freischnitte (9) für die Achsen (7,8) ausgebildet sind.

5. Medizinisches Instrument mit einem Schaft (2), an dessen proximalem Ende eine aus zwei Griffteilen (3a, 3b) bestehende Handhabe (3) angeordnet ist und an dessen distalem Ende ein aus zwei Maulteiten (4a, 4b) bestehendes Werkzeug (4) angeordnet ist, wobei zum Öffnen und Schließen des Werkzeugt (4) mindestens ein Maulteil (4a oder 4b) über eine Zug-/Druckstange (5) gegenüber dem anderen Maulteil (4b oder 4a) um einen von der Stellung der Maulteile (4a,4b) zueinander abhängigen variablen virtuellen Drehpunkt (16) verschwenkbar ist, und wobei die Zug-/Druckstange (5) proximalseitig mit einem verschwenkbar ausgebildeten Griffteil (3b) der Handhabe (3) verbunden ist,
**dadurch gekennzeichnet,**
**dass** beide Maulteile (4a, 4b) über jeweils maximal zwei Anlenkhebel (10,11) verschwenkbar ausgebildet sind, wobei die maximal zwei Anlenkhebel (10, 11) eines jeden Maulteils (4a, 4b) verschwenkbar mit einem Ende ortsfest auf einem am jeweiligen Maulteil (4a oder 4b) angeordneten Zapfen (12) gelagert ist und mit dem anderen Ende verschwenkbar auf einem ortsfest auf der diesem Maulteil (4a, 4b) benachbarten Seite des Schaftes (2) angeordneten Achsstummel (19) gelagert ist.

6. Medizinisches Instrument nach Anspruch 5, **dadurch gekennzeichnet, dass** beide Maulteile (4a, 4b) über jeweils zwei Anlenkhebel (10, 11) verschwenkbar ausgebildet sind. wobei jeder Anlenkhebel (10,11) eines jeden Maulteils (4a, 4b) verschwenkbar mit einem Ende ortsfest auf einem am jeweiligen Maulteil (4a oder 4b) angeordneten Zapfen (12) gelagert ist und mit dem anderen Ende verschwenkbar auf einem ortsfest auf der diesem Maulteil (4a, 4b) benachbarten Seite des Schaftes (2) angeordneten Achsstummel (19) gelagert ist.

7. Medizinisches Instrument nach Anspruch 6, **dadurch gekennzeichnet, dass** der Schnittpunkt (16) der Mittelachsen (17) der Anlenkhebel (10, 11) eines Maulteils (4a, 4b) den Drehpunkt (16) der Maulteile (4a, 4b) bildet.

8. Medizinisches Instrument nach Anspruch 5, **dadurch gekennzeichnet, dass** beide Maulteile (4a, 4b) über jeweils einen Anlenkhebel (10) verschwenkbar ausgebildet sind, wobei jeder Anlenkhebel (10) eines jeden Maulteils (4a, 4b) verschwenkbar mit einem Ende ortsfest auf einem am jeweiligen Maulteil (4a oder 4b) angeordneten Zapfen (12) gelagert ist und mit dem anderen Ende verschwenkbar auf einem ortsfest auf der diesem Maulteil (4a, 4b) benachbarten Seite des Schaftes (2) angeordneten Achsstummel (19) gelagert ist.

9. Medizinisches Instrument nach Anspruch 8, **dadurch gekennzeichnet, dass** in jedem Maulteil (4a, 4b) eine Führungsbahn (20) zur Aufnahme eines an der benachbarten Seite des Schaftes (2) angeordneten Lagerstiftes (21) ausgebildet ist.

10. Medizinisches Instrument nach Anspruch 9, **dadurch gekennzeichnet, dass** der Schnittpunkt (16) der Mittelachse (17) des Anlenkhebels (10) eines Maulteils (4a, 4b) mit der Führungsbahn (20) den Drehpunkt (16) der Maulteile (4a; 4b) bildete.

11. Medizinisches instrument nach einem der Ansprüche 1 bis 10, **dadurch gekennzeichnet, dass** das distale Ende des Schaftes (2) zwei im Wesentlichen parallel zueinander verlaufende Stege (18) umfassend, gabelförmig ausgebildet ist und die proximalen Enden der Maulteile (4a, 4b) zwischen den Stegen (18) gelagert sind.

12. Medizinisches Instrument nach Anspruch 11, **dadurch gekennzeichnet, dass** die Achsen (7, 8) im Bereich der Stege (1,8) im Schaft (2) gelagert sind.

13. Medizinisches Instrument nach einem der Ansprüche 1 bis 12, **dadurch gekennzeichnet, dass** die Zug-/Druckstange (5) distalseitig über jeweils einen Gelenkhebel (6) mit jedem verschwenkbaren Maulteil (4a, 4b) verbunden ist.

14. Medizinisches Instruments nach einem der Ansprüche 1 bis 13, **dadurch gekennzeichnet, dass** das medizinische Instrument (1) ein endoskopisches Instrument ist.

## Claims

1. Medical instrument with a shaft (2) whose proximal end is provided with a handle (3) composed of two grip parts (3a, 3b), and whose distal end is provided with a tool (4) composed of two jaw parts (4a, 4b), in which medical instrument, in order to open and close the tool (4), at least one jaw part (4a or 4b) can be pivoted with respect to the other jaw part (4b or 4a) by means of a push/pull rod (5) and about a virtual rotation point (16) that varies depending on the position of the jaw parts (4a, 4b) relative to each other, and the push/pull rod (5) is connected at the proximal end to a pivotable grip part (3b) of the handle (3), **characterized in that** the two jaw parts (4a, 4b) are mounted pivotably on two axles (7, 8) that are mounted in the shaft (2) and that are transverse to the longitudinal axis (1a) of the instrument, and each jaw part (4a, 4b) is coupled pivotably to the axles (7, 8) via two articulation levers (10, 11).

2. Medical instrument according to Claim 1, **characterized in that** each articulation lever (10, 11) of each jaw part (4a, 4b) is mounted with one end on one of the axles (7, 8) and with the other end pivotably mounted on a pin (12) arranged fixed in position in the respective jaw part (4a, 4b).

3. Medical instrument according to Claim 1 or 2, **characterized in that** the point of intersection (16) of the centre axes (17) of the articulation levers (10, 11) of a jaw part (4a, 4b) forms the rotation point of the jaw parts (4a, 4b).

4. Medical instrument according to one of Claims 1 to 3, **characterized in that** apertures (9) for the axles (7, 8) are formed in the jaw parts (4a, 4b).

5. Medical instrument with a shaft (2) whose proximal end is provided with a handle (3) composed of two grip parts (3a, 3b), and whose distal end is provided with a tool (4) composed of two jaw parts (4a, 4b), in which medical instrument, in order to open and close the tool (4), at least one jaw part (4a or 4b) can be pivoted with respect to the other jaw part (4b or 4a) by means of a push/pull rod (5) and about a virtual rotation point (16) that varies depending on the position of the jaw parts (4a, 4b) relative to each other, and the push/pull rod (5) is connected at the proximal end to a pivotable grip part (3b) of the handle (3), **characterized in that** the two jaw parts (4a, 4b) are pivotable via in each case a maximum of two articulation levers (10, 11), the maximum two articulation levers (10, 11) of each jaw part (4a, 4b) are mounted pivotably, with one end fixed in position on a pin (12) arranged on the respective jaw part (4a or 4b) and with the other end arranged pivotably on an axle neck (19) arranged fixed in position at that end of the shaft (2) adjacent to this jaw part (4a, 4b).

6. Medical instrument according to Claim 5, **characterized in that** the two jaw parts (4a, 4b) are pivotable via in each case two articulation levers (10, 11), each articulation lever (10, 11) of each jaw part (4a, 4b) is mounted pivotably, with one end fixed in position on a pin (12) arranged on the respective jaw part (4a or 4b) and with the other end pivotably mounted on an axle neck (19) arranged fixed in position on that end of the shaft (2) adjacent to this jaw part (4a, 4b).

7. Medical instrument according to Claim 6, **characterized in that** the point of intersection (16) of the centre axes (17) of the articulation levers (10, 11) of a jaw part (4a, 4b) forms the rotation point (16) of the jaw parts (4a, 4b).

8. Medical instrument according to Claim 5, **characterized in that** the two jaw parts (4a, 4b) are pivotable via in each case one articulation lever (10), each articulation lever (10) of each jaw part (4a, 4b) is mounted pivotably, with one end fixed in position on a pin (12) arranged on the respective jaw part (4a or 4b) and with the other end pivotably mounted on an axle neck (19) arranged fixed in position on that end of the shaft (2) adjacent to this jaw part (4a, 4b).

9. Medical instrument according to Claim 8, **characterized in that** a guide track (20) is formed in each jaw part (4a, 4b) and receives a bearing pin (21) arranged on the adjacent side of the shaft (2).

10. Medical instrument according to Claim 9, **characterized in that** the point of intersection (16) of the centre axis (17) of the articulation lever (10) of a jaw part (4a, 4b) forms, with the guide track (20), the rotation point (16) of the jaw parts (4a, 4b).

11. Medical instrument according to one of Claims 1 to 10, **characterized in that** the distal end of the shaft (2) is fork-shaped, with two webs (18) extending substantially parallel to each other, and the proximal ends of the jaw parts (4a, 4b) are mounted between the webs (18).

12. Medical instrument according to Claim 11, **characterized in that** the axles (7, 8) are mounted in the shaft (2) in the area of the webs (18).

13. Medical instrument according to one of Claims 1 to 12, **characterized in that** the push/pull rod (5) is connected at the distal end to each pivotable jaw part (4a, 4b) via a respective toggle lever (6).

14. Medical instrument according to one of Claims 1 to 13, **characterized in that** the medical instrument (1) is an endoscopic instrument.

## Revendications

1. Instrument médical avec une tige (2) à l'extrémité proximale de laquelle est disposée une poignée (3) consistant en deux parties de préhension (3a, 3b) et à l'extrémité distale de laquelle est disposé un outil (4) consistant en deux parties de mâchoire (4a, 4b), où, pour l'ouverture et la fermeture de l'outil (4), au moins une partie de mâchoire (4a ou 4b) est pivotable par le biais d'une barre de traction/compression (5) par rapport à l'autre partie de mâchoire (4b ou 4a) autour d'un centre de rotation (16) virtuel variable dépendant de la position des parties de mâchoire (4a, 4b) l'une par rapport à l'autre et où la barre de traction/compression (5) est reliée du côté proximal à une partie de préhension (3b) de la poignée (3) agencée de manière pivotable,
**caractérisé en ce que**
les deux parties de mâchoire (4a, 4b) agencées de manière pivotable sont montées sur deux axes (7, 8) montés transversalement à l'axe longitudinal (1a) de l'instrument dans la tige (2) et chaque partie de mâchoire (4a, 4b) est couplée avec les axes (7, 8) de manière pivotable par l'intermédiaire de deux leviers articulés (10, 11) à chaque fois.

2. Instrument médical selon la revendication 1 **caractérisé en ce que** chaque levier articulé (10, 11) de chaque partie de mâchoire (4a, 4b) est monté avec une extrémité sur l'un des axes (7, 8) et est monté avec l'autre extrémité pivotable sur un pivot (12) disposé de manière fixe dans la partie de mâchoire (4a, 4b) correspondante.

3. Instrument médical selon la revendication 1 ou 2 **caractérisé en ce que** le point d'intersection (16) des axes (17) des leviers articulés (10, 11) d'une partie de mâchoire (4a, 4b) forme le centre de rotation des parties de mâchoire (4a, 4b).

4. Instrument médical selon l'une des revendications 1 à 3 **caractérisé en ce que** des découpures (9) pour les axes (7, 8) sont formées dans les parties de mâchoire (4a, 4b).

5. Instrument médical avec une tige (2) à l'extrémité proximale de laquelle est disposée une poignée (3) consistant en deux parties de préhension (3a, 3b) et à l'extrémité distale de laquelle est disposé un outil (4) consistant en deux parties de mâchoire (4a, 4b) où, pour l'ouverture et la fermeture de l'outil (4), au moins une partie de mâchoire (4a ou 4b) est pivotable par le biais d'une barre de traction/compression (5) par rapport à l'autre partie de mâchoire (4b ou 4a) autour d'un centre de rotation (16) virtuel variable dépendant de la position des parties de mâchoire (4a, 4b) l'une par rapport à l'autre et où la barre de traction/compression (5) est reliée du côté proximal à une partie de préhension (3b) de la poignée (3) agencée de manière pivotable,
**caractérisé en ce que**
les deux parties de mâchoire (4a, 4b) sont agencées de manière pivotable par l'intermédiaire d'au maximum deux leviers pivotants (10, 11) à chaque fois, où les au maximum deux leviers pivotants (10, 11) de chaque partie de mâchoire (4a, 4b) sont montés de manière pivotable avec une extrémité fixe sur un pivot (12) disposé sur la partie de mâchoire (4a ou 4b) correspondante et avec l'autre extrémité pivotable sur un tourillon (19) disposé de manière fixe sur le côté de la tige (2) voisin de cette partie de mâchoire (4a, 4b).

6. Instrument médical selon la revendication 5 **caractérisé en ce que** les deux parties de mâchoire (4a, 4b) sont agencées de manière pivotable par l'intermédiaire à chaque fois de deux leviers articulés (10, 11), où chaque levier articulé (10, 11) de chaque partie de mâchoire (4a, 4b) est monté de manière pivotable avec une extrémité fixe sur un pivot (12) disposé sur la partie de mâchoire (4a ou 4b) correspondante et avec l'autre extrémité pivotable sur un tourillon (19) disposé de manière fixe sur le côté de la tige (2) voisin de cette partie de mâchoire (4a, 4b).

7. Instrument médical selon la revendication 6 **caractérisé en ce que** le point d'insertion (16) des axes (17) des leviers articulés (10, 11) d'une partie de mâchoire (4a, 4b) forme le centre de rotation (16) des parties de mâchoire (4a, 4b).

8. Instrument médical selon la revendication 5 **caractérisé en ce que** les deux parties de mâchoire (4a, 4b) sont agencées de manière pivotable par l'intermédiaire à chaque fois d'un levier articulé (10), où chaque levier articulé (10) de chaque partie de mâchoire (4a, 4b) est monté de manière pivotable avec une extrémité fixe sur un ergot (12) disposé sur la partie de mâchoire (4a ou 4b) correspondante et avec l'autre extrémité pivotable sur un tourillon (19) disposé de manière fixe sur le côté de la tige (2) voisin de cette partie de mâchoire (4a, 4b).

9. Instrument médical selon la revendication 8 **caractérisé en ce qu'**une glissière de guidage (20) pour recevoir un ergot d'appui (21) disposé sur le côté voisin de la tige (2) est agencée dans chaque partie de mâchoire (4a, 4b).

10. Instrument médical selon la revendication 9 **caractérisé en ce que** le point d'intersection (16) de l'axe (17) du levier articulé (10) d'une partie de mâchoire (4a, 4b) avec la glissière de guidage (20) forment le centre de rotation (16) des parties de mâchoire (4a, 4b).

11. Instrument médical selon l'une des revendications 1 à 10 **caractérisé en ce que** l'extrémité distale de la tige (2) est agencée en forme de fourche comprenant deux branches (18) qui s'étendent sensiblement parallèlement entre elles et les extrémités proximales des parties de mâchoire (4a, 4b) sont montées entre les branches (18).

12. Instrument médical selon la revendication 11 **caractérisé en ce que** les axes (7, 8) sont montés dans la tige (2) dans le domaine des branches (18).

13. Instrument médical selon l'une des revendications 1 à 12 **caractérisé en ce que** la barre de traction/compression (5) est reliée du côté distal à chaque partie de mâchoire (4a, 4b) pivotable par le biais d'un levier articulé (6) à chaque fois.

14. Instrument médical selon l'une des revendications 1 à 13 **caractérisé en ce que** l'instrument médical (1) est un instrument endoscopique.
